(19) **Europäisches Patentamt / European Patent Office / Office européen des brevets**

(11) **EP 4 233 761 B1**

(12) **EUROPEAN PATENT SPECIFICATION**

(45) Date of publication and mention
of the grant of the patent:
**26.03.2025 Bulletin 2025/13**

(21) Application number: **22158812.2**

(22) Date of filing: **25.02.2022**

(51) International Patent Classification (IPC):
**A61B 34/20** $^{(2016.01)}$    **A61B 17/00** $^{(2006.01)}$

(52) Cooperative Patent Classification (CPC):
**A61B 34/20;** A61B 2017/00053; A61B 2017/00243;
A61B 2017/00699; A61B 2017/00703;
A61B 2034/105; A61B 2034/2051; G06N 20/00

(54) **METHOD OF AUTOMATIC REGISTRATION OF A 3D MODEL IN A MEDICAL NAVIGATION SYSTEM DISPLAY DEVICE**

VERFAHREN ZUR AUTOMATISCHEN REGISTRIERUNG EINES 3D-MODELLS IN EINER ANZEIGEVORRICHTUNG EINES MEDIZINISCHEN NAVIGATIONSSYSTEMS

PROCÉDÉ D'ENREGISTREMENT AUTOMATIQUE D'UN MODÈLE 3D DANS UN DISPOSITIF D'AFFICHAGE DE SYSTÈME DE NAVIGATION MÉDICALE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**30.08.2023 Bulletin 2023/35**

(73) Proprietor: **Inheart**
**33600 Pessac (FR)**

(72) Inventors:
• **JUHOOR, Mehdi**
**33600 PESSAC (FR)**
• **PEYRAT, Jean-Marc**
**33600 PESSAC (FR)**

(74) Representative: **Aquinov**
**12, Cours Xavier Arnozan**
**33000 Bordeaux (FR)**

(56) References cited:
**US-A1- 2011 160 574     US-A1- 2016 331 262**
**US-A1- 2018 360 342**

**Description**

The present invention relates to the field of medical image registration. More particularly, the invention relates to a method and a system for registration of a 3D model of an organ of a patient on a medical navigation system display device.

In the context of medical intervention on a patient's organ using catheter, and especially for a catheter cardiac ablation, it is known to prepare the intervention with a virtual 3D model of the organ. For instance, a cardiac ablation can be prepared beforehand by the surgeon, thanks to an identification, being automatic or manual, of ablation targets on a virtual 3D model of the heart.

Such virtual 3D model is generally built from a 3D image of the organ, obtained from a medical imaging method, such as computed tomography or CT-scan, fluoroscopy or MRI. The 3D image is processed to obtain a set of vertices which define a 3D model of the organ. The processing of the 3D image can also highlight features of the organ which can help the surgeon to detect medical intervention targets. For instance, the application EP3622478 from the applicant discloses a method for computing a 3D model of a patient's heart, and wherein the myocardial thickness is modelized to identify low thickness areas, or channels, in the myocardial wall.

Usually, the surgeon then introduces a probe or a catheter into the patient's organ. This catheter allows a navigation system to generate an image of one or more areas of interest of the organ, which is displayed on a screen in order to help the surgeon to assess the position of the targets. To increase accuracy of the intervention, the 3D model on which the medical intervention has been prepared has to be imported into this navigation system.

However, the 3D model must be registered to the reference frame used by the navigation system. In this purpose, a navigation system operator manually moves and rotates the 3D model to match as best as possible the areas of interest generated by the navigation system. Such manual registration is therefore slow, complex and subject to inaccuracy.

US 2018/360342 A1 discloses a method and device that provide improved visualization of soft tissue, such as renal arteries, renal veins and lymph nodes in guiding catheter placement and positioning in the renal region or vasculature. The method and device enable visualization of an electrophysiology catheter application in the renal region which provides for improved imaging of renal structures, including renal arteries, along with one or more adjacent anatomical structures, including renal veins, lymph nodes, other adjacent organs and and/or other adjacent soft tissues that may adversely impact the formation of a lesion during a catheter ablation procedure in or around a renal artery.

US 2016/331262 A1 discloses various embodiments of invasive and non-invasive systems for combined electrophysiological mapping and ablation of a patient's heart. An electrophysiological mapping system (EMS) is configured to operate in conjunction with a cardiac ablation system, which in an invasive embodiment may employ an ablation catheter configured for insertion inside the heart of the patient, and in a non-invasive embodiment may comprise a HIFU transducer. The EMS is programmed and configured to process ECG signals acquired from a patient's torso during the combined electrophysiological mapping and cardiac ablation procedure, and to produce on a display or monitor the real-time or near-real-time voxelmodel-derived visual representation of one or more locations on the patient's heart where at least one scar has been created by the ablation device during the combined procedure.

Considering the above, there is therefore a need for a method of automatic registration of a 3D model of a patient's organ in a reference frame used by a medical navigation system.

The object of the present invention is to answer to this need.

For this purpose, the subject of the invention is a computer-implemented method for displaying a 3D model of an organ of a patient on a navigation system display device according to claim 1.

When a catheter equipped with one or more positioning sensors is introduced into a tubular structure of an organ, the sensor's position is recorded during the motion of the catheter until it reaches its final position. The sensor's position corresponds to spatial coordinates of the catheter's sensor in the patient's frame of reference or coordinates system. As an example, most image-guided ablation procedures involve guiding a catheter into a heart's vessel, such as the coronary sinus. The whole collection of spatial coordinates recorded during the complete motion of the catheter corresponds then to a point cloud representing all or part of the tubular structure. Such tubular structure is also a part of the predefined 3D model of the organ, with which the surgeon has prepared the intervention. Assuming that some vertices of the 3D model have been correctly labelled as belonging to the tubular structure, the tubular structure can be segmented from the 3D model. The invention thus proposes to compute a spatial transformation which, when applied to the 3D model, transforms the 3D model in order to match the 3D model vertices labelled as the tubular structure with the point cloud representing this tubular structure. The result of this spatial transformation on the 3D model is therefore a 3D model automatically registered in the same frame of reference than the spatial coordinates. Consequently, it can be displayed on the navigation system display with the current position of the catheter in a same patient's frame of reference. One can observe that a same registration might be obtained with minimizing a predefined error metric between the result of the reversed spatial transformation one the reference point cloud and the 3D model segmentation.

In the context of the present specification, unless expressly provided otherwise, a "computer" may refer, but is not limited to, a desktop computer, a laptop computer, a tablet computer, a piece of testing equipment, a network appliance, a controller, a digital signal processor, a computational engine within an appliance, a consumer-electronic device, a portable computing device, and/or another electronic device, and/or any combination thereof appropriate to the relevant task at hand. Moreover, the method steps might be all executed on a same device. As a variant, the method steps might be executed on several devices, connected by wires or by a wireless connection.

In the context of the present specification, a "3D model of an organ" is a digital representation of this organ, preferably a mesh, structured or unstructured, comprising a plurality of vertices connected with each other to define a plurality of faces and/or volumes which approximate internal and/or external surfaces and/or volumes of the organ. A 3D model might be a triangle mesh or a quadrilateral mesh, or more generally a polygonal, mesh, which comprises a plurality of vertices, with edges connecting pairs of vertices and/or polygonal faces connecting a closed set of vertices. As a variant, a 3D model might a tetrahedral mesh or a hexahedral mesh or a prism mesh, or more generally a volume mesh, which comprises a plurality of vertices, with polyhedrons connecting a closed set of polygons defined by closed set of vertices. Preferably, each vertex and/or edge and/or polygon and/or polyhedron might be labelled as being a part of a specific sub-part of the organ. A 3D model might be stored as a list of vertices each associated with spatial coordinates and with a set of connections to other vertices, or as a list of vertices associated with spatial coordinates and a list of polygons or faces each defined by a subset of vertices contained in said list of vertices, being understood that any other suitable method of storage might be used in the context of the present specification.

In the context of the present specification, a "spatial transformation" is a geometric transformation which maps an initial set of points to a final set of point and which at least results in a displacement of the initial set of points. It might be a rigid transformation comprising at least a translation or at least a rotation or a combination of one or more rotations with one or more translations. It might also comprise linear or non-linear deformations, as scaling or morphing.

In the context of the present specification, an "error metric" is a metric, or a mathematical function, evaluating the accuracy of a registration of a set of points with a reference set of points. For instance, it might be based on the distance between registered points and reference matching points. In other words, the goal of the spatial transformation computing step is to find the spatial transformation which best aligns the 3D model segmentation with the reference point cloud. As an example, the spatial transformation computing step might comprise a matching points sub-step, which goal is to match all or a part of vertices of the 3D model segmentation with all or part of points of the reference point cloud and a spatial transformation estimation sub-step based on matched points. Theses sub-steps might be implemented only once, with a direct equation solving, or might be iteratively implemented.

According to one embodiment, the landmark displayed on the navigation system might be an image, an icon or a logo displayed at the current sensor position, or a 3D model of the catheter with its tip displayed at the current sensor position. Alternatively or cumulatively, the reference point cloud might be displayed with being superimposed to the result of the computed spatial transformation on the 3D model.

According to one embodiment, the method comprises a preliminary step of acquiring a 3D image and/or recording a 3D image of a patient's organ, particularly his heart or a region of his heart, the 3D model being computed from said 3D image. The 3D image may be acquired directly from an image acquisition device such as a scanner or MRI. Alternatively, the 3D image may be obtained from a recording medium on which it is stored, such as a local memory or a distant database, the 3D image having been acquired beforehand the method.

For example, the step of acquiring the 3D image can be performed by tomography. These techniques are also identified as CT-scan or CAT-scan and are based on the measurement of X-ray absorption by the tissues of an organ. Tomography provides a plurality of 2D images each representing a slice of the organ, which are then combined to reconstruct the 3D image of the anatomical structure of the observed organ. The 3D image comprises a volumetric distribution of pixels, or voxels.

The method according to the invention can thus comprise a 3D modeling step of the 3D image of the organ to generate the 3D model, for instance comprising a modeling at least one layer or one wall of the organ shown in the 3D image, such as an inner face and an outer face of the layer of the organ. Said 3D model might be a mesh, especially a polygonal or a polyhedron mesh, of said layer, especially of said inner and outer faces.

In an example, the method according to the invention can include a step of segmenting one or more regions of interest in the 3D image or 3D model.

According to one embodiment, the method according to the invention comprises a sub-step of segmenting a plurality of volumes in the 3D image or in the 3D model according to their thickness, in particular following a comparison of their thickness to a plurality of thickness levels, each voxel or each vertex being labelled according to the thickness of the volume to which it belongs. Preferably, an image processing method is implemented to detect, segment or extract at least two volumes from the 3D image or from the 3D model by considering for each voxel of the 3D image or for each vertex of the 3D model the thickness of the latter and by comparing this thickness to a maximum threshold or to different thickness levels. For example, the volumes can be segmented according to thickness levels of 1mm, 2mm, 3mm, 4mm and 5mm.

The method of the invention allows to consider for example the wall of the heart, better known as the myocardium. In this case, the 3D image or the 3D model of the heart can be segmented into different volumes according to a myocardial thickness criterion, the 3D model representing therefore a thickness map of the heart.

According to one embodiment, the method according to the invention comprises a step of detecting at least one region of interest in the 3D image or in the 3D model, by identifying for example at least one region, for instance an isthmus in the case of a 3D image or model of a heart, such that it is comprised between two regions of lesser thickness.

Optionally, the method according to the invention comprises a step of classifying each region of interest by means of:

    a. the thickness of this region of interest, compared to the thickness of two adjacent areas,
    b. the width of this region of interest, formed by the spacing of said adjacent zones,
    c. its length, defined by the arrangement of adjacent zones,
    d. the topology of the entrance and/or exit of said region of interest, such as the funnel formed by the two adjacent zones.

In this way, regions of interest can be classified to assist the surgeon in quickly identifying intervention targets. According to one embodiment, several geometric criteria are taken together and combined in order to refine the classification of regions of interest.

Alternatively, the step of detecting a region of interest and classifying this region of interest can be performed, at least partially, manually by a user.

According to one embodiment, the method according to the invention comprises a step of detection and classification of at least one tubular structure in the 3D image or in the 3D model. For instance, in the case of a 3D model of a heart, the method might comprise a step of detection of a vessel, such as a coronary sinus, in the 3D image or in the 3D model.

More precisely, the step of detection and classification of a tubular structure might comprise a manual or an automatic detection of circles and/or of ellipses in at least a subset of 2D images composing the 3D image, each pixel of a 2D image belonging to a detected circle or a detected ellipse being classified as belonging to a detected tubular structure, and the pixels of two 2D images belonging to detected tubular structures with a generally identical shape and/or with generally identical dimensions and/or being generally positioned in the same area in the 2D images being classified as belonging to a same detected tubular structure. Preferably, the step of detection and classification of a tubular structure might comprise an interpolation of the detected tubular structure to the rest of 2D images.

According to the invention, the method comprises a step of identifying one or more areas in the 3D model segmentation and in the reference point cloud having at least one geometrical feature matching a predetermined criteria, and the computed spatial transformation is the spatial transformation which, when applied to the 3D model, minimizes the predefined error metric between the result of this spatial transformation on the 3D model segmentation and the reference point cloud in at least said one or more areas. Considering that tubular structure in which the catheter is inserted might have specific geometrical features, such as a strong curvature and/or a strong torsion, or a specific shape, it is possible to use these geometrical features to enhance the registration.

As an example, said geometrical feature matching a predetermined criteria might be a tubular structure having a curvature value and/or a torsion value exceeding a predetermined threshold or, alternatively or cumulatively, having a shape matching a predetermined shape, such as a cone.

In one embodiment of the invention, the computed spatial transformation is the spatial transformation which, when applied to the 3D model, minimizes the predefined error metric between the result of this spatial transformation on the 3D model segmentation and the reference point cloud only in said one or more identified areas.

In an alternative embodiment, the computed spatial transformation is the spatial transformation which, when applied to the 3D model, minimizes the predefined error metric between the result of this spatial transformation on the 3D model segmentation and the reference point cloud for every points of the 3D model and the reference point cloud, the predefined error metric being weighted with higher weights for points belonging to said one or more identified areas.

According to one embodiment, a value is associated to each point of the 3D model segmentation and/or of the reference point cloud, said value depending on the position of the associated point regarding said one or more areas. In this embodiment, the spatial transformation computing step comprises an iterative estimation of a spatial transformation, which, for each iteration, comprises :

    a. a sub-step of matching each point of at least a part of, especially of a subset of points of, the result of the currently estimated spatial transformation on the 3D model segmentation with one point, notably the closest point, of the reference point cloud, and
    b. a sub-step of adjusting the currently estimated spatial transformation according to the predefined error metric between the result of the currently estimated spatial transformation on the 3D model segmentation and the reference point cloud, wherein the predefined error metric is based on a mean distance value between said matched points, weighted with said associated values.

Said iterative estimation might be implemented as an Iterative Closest Point algorithm, or as a Robust Point Matching algorithm, or any suitable point-set registration algorithm in which the error metric might be weighted. For instance, the predefined error metric might be a Root Mean Square-based, or RMS-based, metric.

According to the invention, the step of receiving a plurality of spatial coordinates comprises the reception of at least a first and a second plurality of spatial coordinates, computed from data received from at least a first and a second sensors of the catheter, notably shifted from each other; and the step of identifying one or more areas in the reference point cloud comprises an estimation of the evolution of the curvature and/or the torsion of the reference point cloud from the first and the second plurality of spatial coordinates.

According to one embodiment, the computed spatial transformation is a square matrix defining at least a rotation and/or a translation. For instance, the matrix might be a 4x4 matrix defining a rotation and a translation. As a variant, the computed spatial transformation might be a vector field, where each vector associates a point from the reference point cloud to a vertex from the 3D model.

According to one embodiment, the method comprises a further step of receiving a set of supplemental local data relative to said organ of the patient from a sensing device distinct of said catheter, and a further step of correcting the computed spatial transformation based on the set of supplemental local data. Thanks to these further steps, the registration might be then corrected based on information about the patient's frame of reference that would be inferred from any new data captured by a sensing device.

Preferably, the step of correcting the computed spatial transformation might be repeated periodically, at any periodic update of the supplemental local data received from the sensing device. It might be implemented after the first computing of the spatial transformation and subsequent to and/or prior to the display step.

By way of example, and without limitation, said supplemental local data might be :

    a. local electrical signals or local electrical information relative to said organ and received, directly or indirectly, from the same catheter, from an other mapping catheter or from an imaging device,
    b. local geometrical features, such as shape or wall thickness, received, directly or indirectly, from an imaging device,
    c. a supplemental plurality of spatial coordinates received, directly or indirectly, from an other catheter equipped with at least one positioning sensor.

According to one embodiment, at least one region of interest, which corresponds to said supplemental local data, can be segmented in the 3D model and the computed spatial transformation is the spatial transformation which, when applied to the 3D model, minimizes the predefined error metric between the result of this spatial transformation on the 3D model segmented region of interest and a reference point cloud associated to said supplemental local data.

For instance, electrical signals on the endocardium captured by a mapping catheter guided in a heart can be used to identify areas of low voltage, slow conduction, or abnormal activity. These areas are correlated with the presence of scars or channels on the heart, which can be segmented on the 3D model of the heart, due to their thickness, width, length and topology. It therefore possible to correct the initial registration achieved with the positioning sensors of the first catheter, with these supplemental information received from this other catheter.

According to one embodiment, the method comprises a further step of periodically receiving spatial coordinates, computed from data received from the at least one sensor of the catheter inside a portion of said organ of the patient; a further step of modeling a periodic motion of said organ of the patient from said periodically received spatial coordinates and a further step of modifying the display positions of the landmark and the result of the computed spatial transformation on the 3D model on the navigation system display device based on said model of the periodic motion of said organ of the patient. A periodic motion of the patient, such as its cardiac and/or respiratory motion, can therefore be compensated to avoid any perturbation in the display of the navigation system.

In a first example, the periodic motion of said organ might be modelized with computing an average set of spatial coordinates from received spatial coordinates over several periodic cycles. As a second example, the modeling of the periodic motion of the organ might be implemented with a machine-learning algorithm, such as a support vector regressor or a relevance vector machine.

According to one embodiment, the method comprises a further step of computing a value relative to an error of registration between the reference point cloud and the result of the computed spatial transformation on the 3D model. Preferably, said value relative to the error of registration might be displayed on the navigation system display device.

Optionally, said value relative to the error of registration might be the value of the predefined error metric between the result of the computed spatial transformation on the 3D model and the reference point cloud.

According to one embodiment, the method comprises a further step of receiving a set of supplemental local data relative to said organ of the patient, computed from data received from at least one sensor of a catheter inside a portion of said organ of the patient, each supplemental local data being associated with spatial coordinates of said sensor, and a further step of displaying each supplemental local data on the result of the computed spatial transformation on the 3D model displayed on the navigation system display device, the displayed position of this supplemental local data being computed from its associated spatial coordinates. It therefore possible to project captured supplemental data on the 3D model displayed on the navigation system, to build a 3D augmented model of the organ.

For instance, each supplemental local data might be displayed with an icon, a marking or a label being superimposed on a vertex of the 3D model, the position of which being determined from the supplemental local data associated spatial coordinates and said computed spatial transformation. As a variant, each supplemental local data might be displayed with a vertex of the 3D model being modified, the position of which being determined from the supplemental local data associated spatial coordinates and said computed spatial transformation, and the color of which being modified depending on the value of the supplemental local data and according to a scalar or color map associating possible values of local data with colors.

By way of example, and without limitation, said supplemental local data might be supplemental electrical local data. Said supplemental electrical local data might be local electrical signals or local electrical information, such as local impedance, relative to said organ and received, directly or indirectly, from an electrode of the same catheter or from an other catheter. As a variant, said supplemental local data might be supplemental mechanical local data. Said supplemental mechanical local data might be local mechanical property of the organ, such as the local elasticity of the tissue, received, directly or indirectly, from a pressure sensor of the same catheter or from an other catheter.

According to one embodiment, the method comprises a further step of computing, for each supplemental local data, a distance between its associated spatial coordinates, and one or more points of the result of the computed spatial transformation on the 3D model, notably a set of points defining a portion of a layer of the organ closest to the associated spatial coordinates, and a further step of computing a value relative to the reliability of this supplemental local data from said computed distance. Thanks to the value relative to their reliability, the supplemental local data can be registered on the 3D model of the organ, but with being weighted with this value relative to their reliability.

The subject of the invention is also a navigation system for intervention assistance according to claim 9.

According to one embodiment, the catheter is a flexible and/or steerable catheter.

According to one embodiment, the navigation system comprises an emitting unit able to emit a magnetic field, and the positioning sensor is a sensor, such as a coil, able to provide a signal in response to said magnetic field traversing the sensor and emitted by the emitting unit, at least one parameter of the signal being a function of a relative location of the sensor regarding the emitting unit. Advantageously, the navigation system comprises a tracking unit configured to compute, from said signal provided by the sensor, a relative location, such as spatial coordinates, of the sensor regarding the emitting unit. Preferably, the collection of all the relative locations computed by the tracking unit during the motion of the catheter forms the plurality of spatial coordinates which is provided to the computing unit.

According to one embodiment, the catheter comprises at least two positioning sensors, shifted from each other. For instance, one positioning sensor might be positioned on a first portion of the catheter and an other positioning sensor might be positioned on a second portion of the catheter, the first and the second portions being steerable from each other.

According to one embodiment, the catheter comprises at least one electrode.

The present invention will be better understood and other advantages will appear on reading the detailed description of an embodiment taken by way of non-limiting example and illustrated by the appended drawings, in which:

Figure 1A is a navigation system for intervention assistance, according to an embodiment of the invention ;

Figure 1B is a catheter of the navigation system shown in Figure 1A ;

Figure 2 is logic flow diagram that depicts a computer-implemented method, according to an embodiment of the invention, for displaying a 3D model of an organ of a patient on the display device of the navigation system shown in Figure 1A ;

Figure 3 shows an initial registration step executed during the method shown in Figure 2 ;

Figure 4 shows the image displayed on the display device of the navigation system shown in Figure 1A after the execution of the initial registration step shown in Figure 3 ;

Figure 5 shows a registration correction step executed during the method shown in Figure 2 ; and

Figure 6 shows the image displayed on the display device of the navigation system shown in Figure 1A after the execution of an electrical data projection step of the method shown in Figure 2.

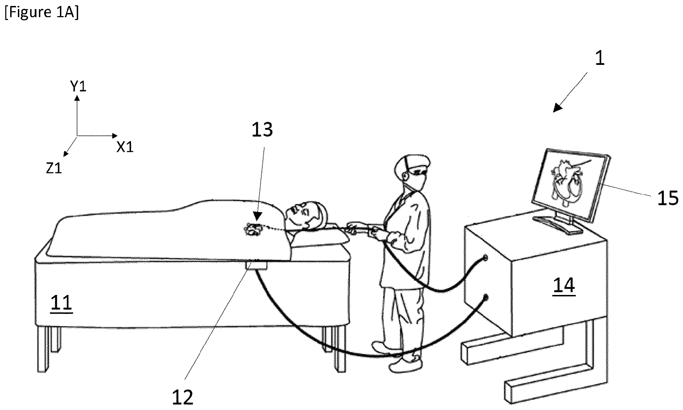

Reference is made to Figure 1A that shows a navigation system 1 for intervention assistance, and especially for catheter heart ablation assistance.

The system 1 includes a procedural table 11 on which a patient is lying. The system 1 also includes an emitting unit, or a field generator, 12 mounted to or provided adjacent the table 11, e.g., which is positioned and/or oriented to generate an electromagnetic field encompassing a patient's organ, such as a patient's heart. The system 1 also includes a catheter 13, which may be introduced into the patient's organ, e.g., into the patient's heart.

The electromagnetic field generated by the emitting unit 12 provides an external reference frame X1, Y1, Z1, which is substantially stationary relative to the table 11 and within which the catheter 13 may be located, after insertion in the patient's organ.

The system 1 includes a controller 14 that communicates with the emitting unit 12 and/or the catheter 13 introduced into the patient's body, e.g., to identify the location of one or more sensors on the catheter 13 relative to the external reference frame.

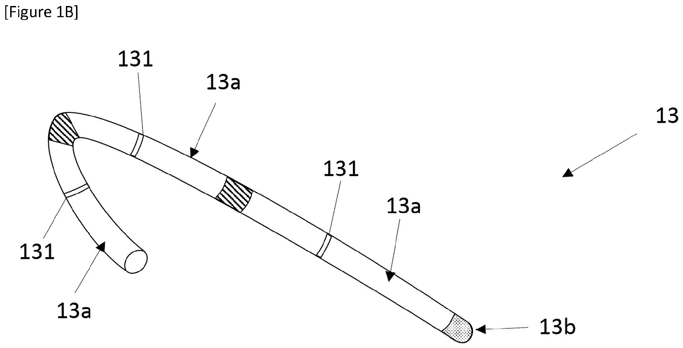

Turning to Figure 1B, the catheter 13 includes a plurality of sensors 131 whose location may be identified by a tracking unit of the controller 14.

In the shown example, the catheter 13 comprises an elongate tubular member including a plurality of steerable or flexible portions 13a and a distal end 13b sized for introduction into a patient's tubular structure, such a coronary sinus or a great cardiac vein. The catheter 13 may include a handle or hub on a proximal end, e.g., which might include one or more actuators, connectors, and the like.

Each portion 13a may be flexible and/or steerable to facilitate the guiding of the distal portion 13b within the patient's tubular structure. For instance, each portion 13a may include one or more steering elements, operable from the catheter handle, to control the bending of this portion from a generally straight configuration to a curved configuration. The catheter 13 can be therefore bent to follow the general curvature of the patient's tubular structure.

Each portion 13a of the catheter 13 comprises at least one positioning sensor 131, which might be an antennae or a coil reactive to the electromagnetic field generated by the emitting unit 12. Each sensor 131 might be coupled to the tracking unit of the controller 14 via one or more leads (not shown) extending in a lumen of the catheter.

In the shown example, each sensor 131 may be an at least three degree of freedom sensor, i.e., providing signals corresponding to a coordinate position of the sensor 131 in three-dimensional space within the reference frame X1, Y1, Z1. In an alternative embodiment, one or more sensors 131 may be six degree of freedom sensor which also provides signals relative a rotational position of the sensor relative to the external reference frame. Further, any number of additional sensors capable of location within a predetermined number of degrees of freedom may be provided on each portion of the catheter.

The tracking unit of the controller 14 receives signals provided by each sensor 131 and is configured to compute, from each received signal, spatial coordinates of a location of the corresponding sensor 131 within the reference frame X1, Y1, Z1.

The system 1 also comprises a display device 15, which the controller 13 controls to present information to a surgeon to provide an assistance to the intervention.

As the catheter 13 contacts or comes within proximity of the tubular structure, the controller 14 may register points in the external reference frame, from sensors 131 locations computed by the tracking unit, and combine all the points to create a spatial representation of the tubular structure, which may be presented on the display device 15.

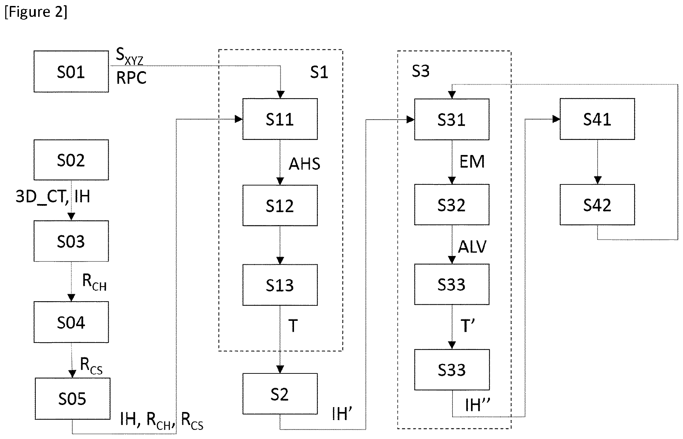

Reference is now made to Figure 2 which shows a computer-implemented method for displaying a 3D model of an organ of a patient on the display device 15, and which is executed by a computing unit of the controller .13.

As a preliminary step S01, the catheter 13 has been introduced into the patient's heart, through the coronary sinus CS, and the location of each sensor 131 has been computed by the tracking unit of the controller 14 during the motion of the catheter 13 to its current position. The set of locations computed by the tracking unit during the motion of the catheter forms a plurality of spatial coordinates $S_{XYZ}$ which is provided to the computing unit of the controller 13.

As described previously, one can note that this plurality of spatial coordinates $S_{XYZ}$ defines a reference point cloud RPC representing all or part of the coronary sinus CS.

In an other preliminary step 502, a 3D image 3D_CT of the patient's heart has been acquired from a CT-scan method, and a 3D model IH representing a thickness map of the patient's heart myocardium has been generated from the 3D image.

More precisely, an inner surface and an outer surface of the patient's myocardium have been modelized from the 3D image 3D_CT with defining a plurality of vertices to form a mesh. Moreover, a plurality of volumes in the resulting model IH have been segmented according to their thickness with each vertex being labelled according to the thickness of the volume to which it belongs. The 3D model IH has its own frame of reference X2, Y2, Z2, which might be completely different from the frame of reference X1, Y1, Z1.

In a further preliminary step 503, at least one region $R_{CH}$, has been detected and segmented in the 3D model IH as being comprised between two regions of lesser thickness and has been classified as an ablation target, taking into account its thickness, its width, its length and its entrance and exit topologies. The resulting 3D model IH may therefore have helped the surgeon to prepare the catheter ablation intervention.

In an other further preliminary step 504, a region $R_{CS}$ has been detected and segmented in the 3D model IH as being a tubular structure and has been classified as being all or part of the coronary sinus CS.

One can note that the step S02 to S04 might be executed by an other computing unit than the controller 14, and that steps S03 and/or S04 might be executed fully automatically by this computing or with a human assistance. The resulting 3D model IH might be stored on a recording medium, such as a memory of the controller 14 or an external memory support or a distant database through which it is accessible to the controller 14.

In a step 505, the 3D model IH in the frame of reference X2, Y2, Z2, with segmented regions $R_{CH}$ and $R_{CS}$, is provided to the computing unit of the controller 14.

In a step S1, a spatial transformation T, allowing to register the 3D model IH in the frame of reference X1, Y1, Z1 of the reference point cloud RPC, is computed by the controller 14.

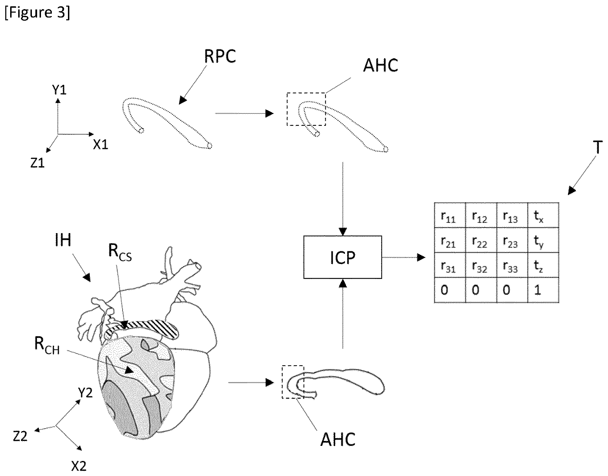

The execution of the step S1 has been shown on Figure 3.

The step S1 comprises a sub-step S11 consisting in the identification of one or more areas in the region $R_{CS}$ of the 3D model IH and in the reference point cloud RPC having at least one geometrical feature matching a predetermined criteria. In the example shown, the sub-step S11 consists in identifying areas AHC of the coronary sinus CS represented by the region $R_{CS}$ and by the reference point cloud RPC which have a curvature and/or a torsion exceeding a predetermined threshold.

Considering reference point cloud $R_{PC}$, since all the sensors 131 of the catheter 13 have been guided consecutively into the coronary sinus CS, the curvature and the torsion of the reference point cloud RPC can be estimated by controller 14 with comparing the evolution of the vectors connecting the locations of each pair of sensors 131 during the catheter motion into the coronary sinus CS.

Considering the region $R_{CS}$ of the 3D model IH, algorithms for curvature extraction and/or torsion extraction from a tubular 3D model, consisting in extracting one ore more curves from a point cloud and then in computing the curvature of this/these curves, are known per se and will not be developed furthermore. Such algorithms might also be applied to the reference point cloud $R_{PC}$ to evaluate the curvature and/or the torsion of the reference point cloud $R_{PC}$.

Areas AHC corresponding to coronary sinus portions with a strong curvature and/or a strong torsion can be therefore segmented from both the region $R_{CS}$ of the 3D model IH and the reference point cloud RPC. Alternatively or cumulatively, other areas with specific geometrical features, such as a predefined shape, might also be segmented.

In a sub-step S12, weights are attributed to each vertex of the region $R_{CS}$ of the 3D model IH and to each point of the reference point cloud RPC, the weights attributed to vertices and points belonging to areas AHC being higher to the weights for other vertices and points.

In a sub-step S13, an Iterative Closest Point algorithm is executed by the controller 14 to iteratively estimate a spatial transformation T which, when applied to the 3D model IH, minimizes an error metric E between the result $T(R_{CS})$ of this spatial transformation T on the region $R_{CS}$ and the reference point cloud RPC, the predefined error metric taking into account the weights attributed to the vertices and points in the sub-step S12.

For instance, the predefined error metric E might be computed with the following equation:

$$(1)\ E = \sqrt{\frac{1}{\sum_{i=1}^{N}\omega_i}\sum_{i=1}^{N}\omega_i\left\|RPC_i, T\left(R_{CS,i}\right)\right\|^2}$$

where E is a root mean square-based error metric, N is the number of points of the reference point cloud RPC which match points of the region $R_{CS}$, $RPC_i$ and $R_{CS,i}$ are two matched points of the reference point cloud RPC and the region $R_{CS}$, $\omega_i$ is the weight attributed to these points, T is the estimated spatial transformation to be applied to the 3D model IH, and $\|x,y\|$ is the distance between two points x and y.

In the shown example, the spatial transformation T which is estimated by the Iterative Closest Point (ICP) algorithm is constrained to a rigid transformation and is defined by a 4x4 matrix defining a rotation and a translation.

Despite the fact that the detection of areas AHC and the attribution of weights to points strongly enhance the registration, sub-steps S11 and S12 might be skipped and the spatial transformation might be directly computed by the controller 14 at the step S13, from all the points of the reference point cloud RPC and the region $R_{CS}$ and with a non-weighted error metric. As an other alternative, the sub-step S12 only might be skipped, and the spatial transformation might be computed by the controller 14 at the step S13, from the sole points of the reference point cloud RPC and the region $R_{CS}$ which belong to the areas AHC, and with a non-weighted error metric.

One can note that the invention is not limited to the implementation of an Iterative Closest Point algorithm and that other suitable point-set registration algorithm, especially in which the error metric might be weighted, can be executed by the controller 14 for the implementation of the sub-step S13.

In a step S2, a registered 3D model IH' is computed by the controller 14, with applying the estimated spatial transformation T to the whole 3D model IH. This registered 3D model IH' is then displayed on the display device 15, simultaneously to a landmark LM positioned, in the reference frame X1, Y1, Z1, at a current position of the catheter 13.

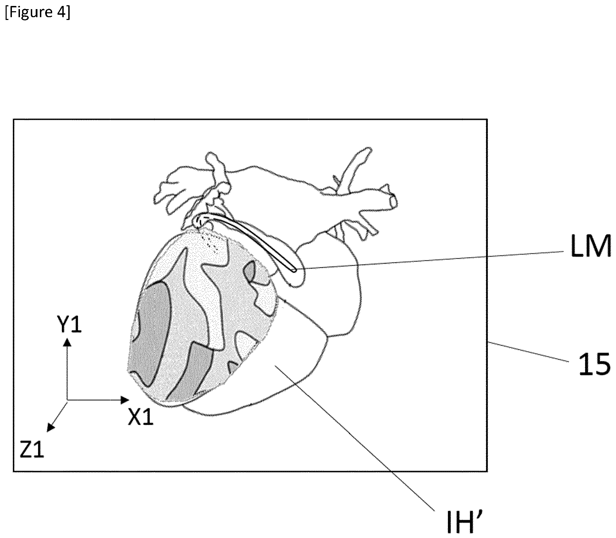

Reference is made to the Figure 4, which shows a simplified view of the display device 15 at the end of the step S2. In the depicted embodiment, the landmark LM is a 3D model of the catheter 13, represented in its current configuration and its current location in the reference frame X1, Y1, Z1.

Optionally, the value of the error metric E between the result of the computed spatial transformation T on the 3D model IH and the reference point cloud RPC might be displayed on the device 15, as a value relative to an error of registration.

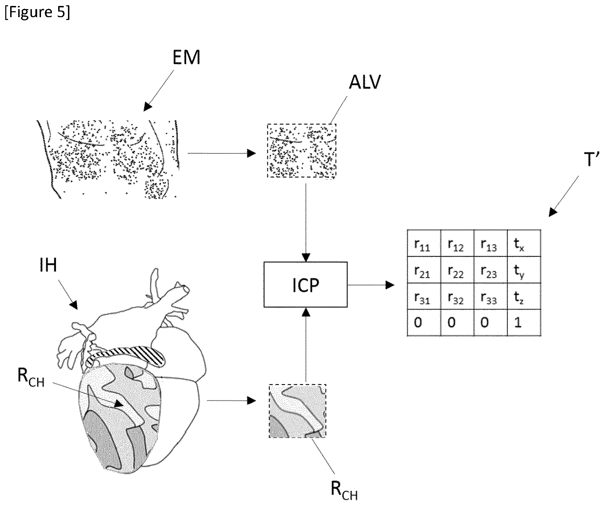

Steps S1 and S2 corresponds to an initial registration of the 3D model IH in the reference frame X1, Y1, Z1. However, this initial registration might be subject to slight errors, which can be corrected in a real-time correction step S3 described hereinafter. Reference is also made to Figure 5 which depicts the execution of this step.

During the intervention, in a sub-step S31, a second catheter (not represented) has been introduced into the patient's heart. This second catheter might be mapping catheter provided with one or more electrodes. These electrodes provide periodically to the controller 14 local electrical signals, captured on the endocardium and each associated with spatial coordinates, from which the controller 14 might generate an electrophysiological map EM of the heart, such as peak-to-peak voltage map. In a sub-step S32, area ALV of low voltage, slow conduction, or abnormal activity can be segmented, by way of known methods executed by the controller 14, from the map EM.

This area ALV can be correlated to region $R_{CH}$, which has been detected and segmented in the 3D model IH, and which corresponds to a myocardium channel responsible of arrythmias.

Similarly to step S13, in a sub-step S33, a point-set registration algorithm might be executed by the controller 14, from the area ALV and the region $R_{CH}$, to correct the spatial transformation T in a corrected spatial transformation T' which, when applied to the 3D model IH, minimizes an error metric E between the result $T(R_{CH})$ of this spatial transformation T on the region $R_{CH}$ and the area ALV.

Moreover, in a step S34, the registered 3D model IH" is computed again by the controller 14, with applying the estimated spatial transformation T' to the whole 3D model IH. This registered 3D model IH" is updated on the display device 15.

Steps S31 to S34 might be repeated periodically, for any periodic update of the electrophysiological map EM.

One can note that the invention is not limited to the use of local electrical signals provided by a mapping catheter to correct the spatial transformation T, but can be extended to the use of other kind of local data which might be associated to locations in the frame of reference X1, Y1, Z1, such as local shape or local wall thickness, received, directly or indirectly, from an imaging device, or spatial coordinates received, directly or indirectly, from an other catheter equipped with at least one positioning sensor.

In a further step S41, each electrical local data received from the mapping catheter, or from electrodes provided to the catheter 13, is displayed on the result IH" of the corrected spatial transformation T' on the 3D model IH displayed on the display device 15.

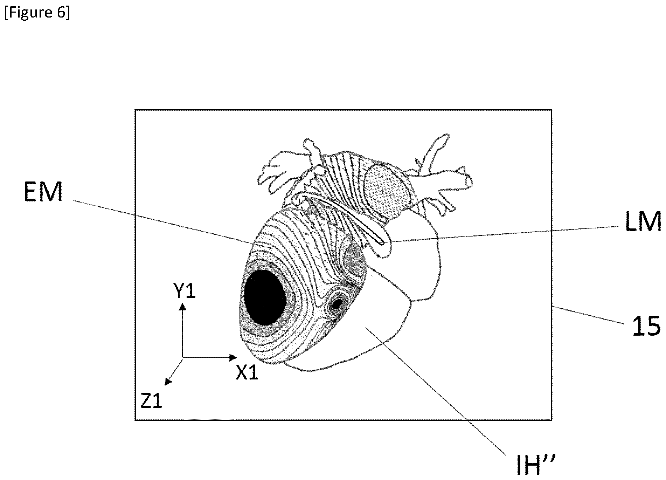

As depicted on Figure 6, which shows a later simplified view of the display device 15, each electrical local data might be represented on a vertex of the 3D model IH" whose location corresponds to the spatial coordinates of this electrical local data, and whose color depends on the value of the electrical local data, according to a color map associating possible electrical values with colors.

Optionally, in a further step S42, for each electrical local data, the controller 14 computes a distance between its spatial coordinates, and a set of vertices of the 3D model IH" which defines a portion of the inner or the outer surface of the myocardium which is closest to these spatial coordinates. Said distance might be converted by the controller 14 in a value relative to the reliability of this electrical local data, said value relative to their reliability being displayed on the display device 15. For instance, the value relative to the reliability might be obtained from a transfer function taking as an input the distance computed from spatial coordinates associated to a local data and outputting a value comprised between 0 and 1, where 0 indicates that the local data is not reliable at all and where 1 indicates that the local data is fully reliable. Such transfer function might be a step function, a rectangular function, a ramp function, a triangle function, a sigmoid or logistic function or a gaussian function.

Steps S41 and S42 might be repeated, for any new electrical data received from a catheter.

The methods disclosed herein may also be implemented by software programs executable by a computer system. Further, implementations may include distributed processing and parallel processing, especially for processing in parallel several or all data in the data sets.

The illustrations described herein are intended to provide a general understanding of the structure of various embodiments. These illustrations are not intended to serve as a complete description of all of the elements and features of apparatus, processors and systems that utilizes the structures or methods described therein. Many other embodiments or combinations thereof may be apparent to those of ordinary skills in the art upon reviewing the disclosure by combining the disclosed embodiments. Other embodiments may be utilized and derived from the disclosure, such that structural and logical substitutions and changes may be made without departing from the scope of the disclosure.

Further, the disclosure and the illustrations are to be considered as illustrative rather than restrictive. Thus, the scope of the following claims is to be determined by the broadest permissible interpretation of the claims and shall not be restricted or limited by the foregoing description.

**Claims**

1. A computer-implemented method for displaying a 3D model of an organ of a patient on a navigation system display device (15), the method comprising :

   a. (S01) Receiving a plurality of spatial coordinates ($S_{XYZ}$), computed from data received from at least one sensor (131) of a catheter (13) inside a portion (CS) of said organ of the patient; the plurality of spatial coordinates defining a reference point cloud (RPC) representing said portion of said organ of the patient;
   b. (S05) Receiving a plurality of points, said plurality of points defining a predefined 3D model (IH) of said organ of the patient, a sub-plurality of points being labelled to define a 3D model segmentation ($R_{CS}$) corresponding to said portion of said organ of the patient ;
   c. (S1) Computing a spatial transformation (T) which, when applied to the 3D model, minimizes a predefined error metric (E) between the result ($T(R_{CS})$) of this spatial transformation on the 3D model segmentation and the reference point cloud;
   d. (S2) Displaying on the navigation system display device a landmark (LM) at a current position of the catheter obtained from said plurality of spatial coordinates and, simultaneously, the result (IH') of the computed spatial transformation on the 3D model
   wherein the method comprises a step (S11) of identifying one or more areas (AHC) in the 3D model segmentation ($R_{CS}$) and in the reference point cloud (RPC) having at least one geometrical feature matching a predetermined criteria, and the computed spatial transformation (T) is the spatial transformation which, when applied to the 3D model (IH), minimizes the predefined error metric (E) between the result ($T(R_{CS})$) of this spatial transformation on the 3D model segmentation and the reference point cloud in at least said one or more areas ;
   **characterised in that**
   the step (S01) of receiving a plurality of spatial coordinates ($S_{XYZ}$) comprises the reception of at least a first and a second plurality of spatial coordinates, computed from data received from at least a first and a second sensors (131) of the catheter (13); and wherein the step (S11) of identifying one or more areas (AHC) in the reference point cloud (RPC) comprises an estimation of the evolution of the curvature and/or the torsion of the reference point cloud from the first and the second plurality of spatial coordinates.

2. The method according to claim 1, wherein a value is associated to each point of the 3D model segmentation ($R_{CS}$) and/or of the reference point cloud (RPC), said value depending on the position of the associated point regarding said one or more areas (AHC), wherein the spatial transformation computing step (S1) comprises an iterative estimation (S13) of a spatial transformation (T), which, for each iteration, comprises :

   a. a sub-step of matching each point of at least a part of the result ($T(R_{CS})$) of the currently estimated spatial transformation on the 3D model segmentation with one point of the reference point cloud, and
   b. a sub-step of adjusting the currently estimated spatial transformation according to the predefined error metric (E) between the result of the currently estimated spatial transformation on the 3D model segmentation and the reference point cloud, wherein the predefined error metric is based on a mean distance value between said matched points, weighted with said associated values.

3. The method according to one of the preceding claims, wherein the computed spatial transformation (T) is a square matrix defining at least a rotation and/or a translation.

4. The method according to one of the preceding claims, the method comprising a further step (S31) of receiving a set of supplemental local data relative to said organ of the patient from a sensing device distinct of said catheter, and a further step (S33) of correcting the computed spatial transformation (T) based on the set of supplemental local data.

5. The method according to one of the preceding claims, the method comprising a further step of periodically receiving spatial coordinates ($S_{XYZ}$), computed from data received from the at least one sensor (131) of the catheter (13) inside a portion (CS) of said organ of the patient; a further step of modeling a periodic motion of said organ of the patient from said periodically received spatial coordinates and a further step of modifying the display positions of the landmark (LM) and the result (IH') of the computed spatial transformation (T) on the 3D model (IH) on the navigation system display device (15) based on said model of the periodic motion of said organ of the patient.

6. The method according to one of the preceding claims, the method comprising a further step of computing a value (E) relative to an error of registration between the reference point cloud (RPC) and the result (IH') of the computed spatial transformation (T) on the 3D model (IH).

**7.** The method according to one of the preceding claims, the method comprising a further step (S31) of receiving a set of supplemental local data relative to said organ of the patient, computed from data received from at least one sensor of a catheter inside a portion of said organ of the patient, each supplemental local data being associated with spatial coordinates of said sensor, and a further step (S41) of displaying each supplemental local data on the result (IH') of the computed spatial transformation (T) on the 3D model (IH) displayed on the navigation system display device (15), the displayed position of this supplemental local data being computed from its associated spatial coordinates.

**8.** The method according to the preceding claim, the method comprising a further step (S42) of computing, for each supplemental local data, a distance between its associated spatial coordinates, and one or more points of the result (IH') of the computed spatial transformation (T) on the 3D model (IH), and a further step of computing a value relative to the reliability of this supplemental local data from said computed distance.

**9.** Navigation system (1) for intervention assistance, the navigation system comprising at least one catheter (13) comprising at least one positioning sensor (131), a display (15), and a computing unit (14) designed to implement the method according to one of the preceding claims, and designed to control the display of said landmark (LM) and said result (IH') of the computed spatial transformation (T) on the 3D model (IH) on said display.

**Patentansprüche**

**1.** Computerimplementiertes Verfahren zum Anzeigen eines 3D-Modells eines Organs eines Patienten auf einer Anzeigevorrichtung (15) eines Navigationssystems, das Verfahren umfassend:

a. (S01) Empfangen einer Vielzahl von räumlichen Koordinaten ($S_{XYZ}$), die aus Daten berechnet ist, die von mindestens einem Sensor (131) eines Katheters (13) im Inneren eines Abschnitts (CS) des Organs des Patienten empfangen werden; die Vielzahl der räumlichen Koordinaten eine Referenzpunktwolke (RPC) definiert, die den Abschnitt des Organs des Patienten darstellt;

b. (S05) Empfangen einer Vielzahl von Punkten, wobei die Vielzahl von Punkten ein vordefiniertes 3D-Modell (IH) des Organs des Patienten definiert, wobei eine Untervielzahl von Punkten markiert ist, um eine 3D-Modellsegmentierung (Rcs) zu definieren, die dem Abschnitt des Organs des Patienten entspricht;

c. (S1) Berechnen einer räumlichen Transformation (T), die, wenn sie auf das 3D-Modell angewendet wird, eine vordefinierte Fehlermetrik (E) zwischen dem Ergebnis (T(Rcs)) dieser räumlichen Transformation auf die 3D-Modellsegmentierung und die Referenzpunktwolke minimiert;

d. (S2) Anzeigen eines Orientierungspunkts (LM) an einer aktuellen Position des Katheters, der aus der Vielzahl von räumlichen Koordinaten erhalten wird, und, gleichzeitig, des Ergebnisses (IH') der berechneten räumlichen Transformation auf dem 3D-Modell auf der Anzeigevorrichtung des Navigationssystems,

wobei das Verfahren einen Schritt (S11) eines Identifizierens eines oder mehrerer Bereiche (AHC) in der 3D-Modellsegmentierung (Rcs) und in der Referenzpunktwolke (RPC) umfasst, die mindestens ein geometrisches Merkmal aufweisen, das mit einem zuvor bestimmten Kriterium übereinstimmt, und die berechnete räumliche Transformation (T) die räumliche Transformation ist, die, wenn sie auf das 3D-Modell (IH) angewendet wird, die vordefinierte Fehlermetrik (E) zwischen dem Ergebnis (T(Rcs)) dieser räumlichen Transformation auf die 3D-Modellsegmentierung und die Referenzpunktwolke in mindestens einem oder mehreren Bereichen minimiert;

**dadurch gekennzeichnet, dass** der Schritt (S01) des Empfangens einer Vielzahl von räumlichen Koordinaten ($S_{XYZ}$) den Empfang von mindestens einer ersten und einer zweiten Vielzahl von räumlichen Koordinaten umfasst, die aus Daten berechnet werden, die von mindestens einem ersten und einem zweiten Sensor (131) des Katheters (13) empfangen werden, und wobei der Schritt (S11) des Identifizierens eines oder mehrerer Bereiche (AHC) in der Referenzpunktwolke (RPC) eine Schätzung der Entwicklung der Krümmung und/oder der Torsion der Referenzpunktwolke aus der ersten und der zweiten Vielzahl von räumlichen Koordinaten umfasst.

**2.** Verfahren nach Anspruch 1, wobei jeder Punkt der 3D-Modellsegmentierung (Rcs) und/oder die Referenzpunktwolke (RPC) mit einem Wert verknüpft ist, wobei der Wert von der Position des verknüpften Punkts in Bezug auf den einen oder die mehreren Bereiche (AHC) abhängt, wobei der Berechnungsschritt (S1) der räumlichen Transformation eine iterative Schätzung (S13) einer räumlichen Transformation (T) umfasst, die, für jede Iteration, umfasst:

a. ein Unterschritt eines Übereinstimmens jedes Punkts von mindestens einem Teil des Ergebnisses (T(Rcs)) der aktuell geschätzten räumlichen Transformation auf der 3D-Modellsegmentierung mit einem Punkt der Referenzpunktwolke und

b. einen Unterschritt eines Anpassens der aktuell geschätzten räumlichen Transformation gemäß der vordefinierten Fehlermetrik (E) zwischen dem Ergebnis der aktuell geschätzten räumlichen Transformation auf der 3D-Modellsegmentierung und der Referenzpunktwolke, wobei die vordefinierte Fehlermetrik auf einem mittleren Distanzwert zwischen den übereinstimmten Punkten basiert, gewichtet mit den verknüpften Werten.

3. Verfahren nach einem der vorstehenden Ansprüche, wobei die berechnete räumliche Transformation (T) eine quadratische Matrix ist, die mindestens eine Rotation und/oder eine Translation definiert.

4. Verfahren nach einem der vorstehenden Ansprüche, das Verfahren einen umfassend weiteren Schritt (S31) des Empfangens eines Satzes ergänzender lokaler Daten bezüglich des Organs des Patienten von einer Sensorvorrichtung, die von dem Katheter verschieden ist, und einen weiteren Schritt (S33) eines Korrigierens der berechneten räumlichen Transformation (T) basierend auf dem Satz ergänzender lokaler Daten.

5. Verfahren nach einem der vorstehenden Ansprüche, das Verfahren umfassend einen weiteren Schritt des periodischen Empfangens räumlicher Koordinaten ($S_{XYZ}$), berechnet aus Daten, die von mindestens einem Sensor (131) des Katheters (13) im Inneren eines Abschnitts (CS) des Organs des Patienten empfangen wird; einen weiteren Schritt eines Modellierens einer periodischen Bewegung des Organs des Patienten aus den periodisch empfangenen räumlichen Koordinaten und einen weiteren Schritt eines Modifizierens der Anzeigepositionen des Orientierungspunkts (LM) und des Ergebnisses (IH') der berechneten räumlichen Transformation (T) auf dem 3D-Modell (IH) auf der Anzeigevorrichtung (15) des Navigationssystems basierend auf dem Modell der periodischen Bewegung des Organs des Patienten.

6. Verfahren nach einem der vorstehenden Ansprüche, das Verfahren umfassend einen weiteren Schritt des Berechnens eines Werts (E) bezüglich eines Registrierungsfehlers zwischen der Referenzpunktwolke (RPC) und dem Ergebnis (IH') der berechneten räumlichen Transformation (T) auf dem 3D-Modell (IH).

7. Verfahren nach einem der vorstehenden Ansprüche, das Verfahren umfassend einen weiteren Schritt (S31) des Empfangens eines Satzes ergänzender lokaler Daten bezüglich des Organs des Patienten, die aus Daten berechnet werden, die von mindestens einem Sensor eines Katheters im Inneren eines Abschnitts des Organs des Patienten empfangen werden, wobei alle ergänzenden lokalen Daten mit räumlichen Koordinaten des Sensors verknüpft sind, und einen weiteren Schritt (S41) des Anzeigens aller ergänzenden lokalen Daten zu dem Ergebnis (IH') der berechneten räumlichen Transformation (T) auf dem 3D-Modell (IH), das auf der Anzeigevorrichtung (15) des Navigationssystems angezeigt wird, wobei die angezeigte Position dieser ergänzenden lokalen Daten aus ihren verknüpften räumlichen Koordinaten berechnet wird.

8. Verfahren nach dem vorstehenden Anspruch, das Verfahren umfassend einen weiteren Schritt (S42) des Berechnens, für alle zusätzlichen lokalen Daten, einer Distanz zwischen den verknüpften räumlichen Koordinaten und einem oder mehreren Punkten des Ergebnisses (IH') der berechneten räumlichen Transformation (T) auf dem 3D-Modell (IH) und einen weiteren Schritt des Berechnens eines Werts bezüglich der Zuverlässigkeit dieser zusätzlichen lokalen Daten aus der berechneten Distanz.

9. Navigationssystem (1) für eine Interventionsunterstützung, das Navigationssystem umfassend mindestens einen Katheter (13), umfassend mindestens einem Positionierungssensor (131), eine Anzeige (15) und eine Recheneinheit (14), die ausgebildet ist, um das Verfahren nach einem der vorstehenden Ansprüche zu implementieren, und ausgebildet ist, um die Anzeige des Orientierungspunkts (LM) und des Ergebnisses (IH') der berechneten räumlichen Transformation (T) auf dem 3D-Modell (IH) auf der Anzeige zu steuern.

## Revendications

1. Procédé implémenté par ordinateur permettant d'afficher un modèle 3D d'un organe d'un patient sur un dispositif d'affichage de système de navigation (15), le procédé comprenant :

a. (S01) la réception d'une pluralité de coordonnées spatiales ($S_{XYZ}$), calculées à partir de données reçues d'au moins un capteur (131) d'un cathéter (13) à l'intérieur d'une portion (CS) dudit organe du patient ; la pluralité de coordonnées spatiales définissant un nuage de points de référence (RPC) représentant ladite portion dudit organe du patient ;

b. (S05) la réception d'une pluralité de points, ladite pluralité de points définissant un modèle 3D prédéfini (IH) dudit organe du patient, une sous-pluralité de points étant marquée pour définir une segmentation de modèle 3D (Rcs) correspondant à ladite portion dudit organe du patient ;

c. (S1) le calcul d'une transformation spatiale (T) qui, lorsqu'elle est appliquée au modèle 3D, minimise une métrique d'erreur prédéfinie (E) entre le résultat (T(Rcs)) de cette transformation spatiale sur la segmentation de modèle 3D et le nuage de points de référence ;

d. (S2) l'affichage sur le dispositif d'affichage de système de navigation d'un repère anatomique (LM) au niveau d'une position actuelle du cathéter, obtenue à partir de ladite pluralité de coordonnées spatiales et, simultanément, le résultat (IH') de la transformation spatiale calculée sur le modèle 3D

dans lequel le procédé comprend une étape (S11) d'identification d'une ou plusieurs zones (AHC) dans la segmentation de modèle 3D (Rcs) et dans le nuage de points de référence (RPC) ayant au moins une caractéristique géométrique concordant avec un critère prédéterminé, et la transformation spatiale calculée (T) est la transformation spatiale qui, lorsqu'elle est appliquée au modèle 3D (IH), minimise la métrique d'erreur prédéfinie (E) entre le résultat (T(Rcs)) de cette transformation spatiale sur la segmentation de modèle 3D et le nuage de points de référence au moins dans ladite ou lesdites zones ;

**caractérisé en ce que** l'étape (S01) de réception d'une pluralité de coordonnées spatiales ($S_{XYZ}$) comprend la réception d'au moins une première et une seconde pluralité de coordonnées spatiales, calculées à partir de données reçues au moins de premier et second capteurs (131) du cathéter (13) ; et dans lequel l'étape (S11) d'identification d'une ou plusieurs zones (AHC) dans le nuage de points de référence (RPC) comprend une estimation de l'évolution de la courbure et/ou de la torsion du nuage de points de référence par rapport aux première et seconde pluralités de coordonnées spatiales.

2. Procédé selon la revendication 1, dans lequel une valeur est associée à chaque point de la segmentation de modèle 3D (Rcs) et/ou du nuage de points de référence (RPC), ladite valeur dépendant de la position du point associé concernant ladite ou lesdites zones (AHC), dans lequel l'étape de calcul de transformation spatiale (S1) comprend une estimation itérative (S13) d'une transformation spatiale (T), qui, pour chaque itération, comprend :

a. une sous-étape de concordance de chaque point d'au moins une partie du résultat (T(Rcs)) de la transformation spatiale actuellement estimée sur la segmentation de modèle 3D avec un point du nuage de points de référence, et

b. une sous-étape d'ajustement de la transformation spatiale actuellement estimée selon la métrique d'erreur prédéfinie (E) entre le résultat de la transformation spatiale actuellement estimée sur la segmentation de modèle 3D et le nuage de points de référence, dans lequel la métrique d'erreur prédéfinie est en fonction d'une valeur de distance moyenne entre lesdits points concordants, pondérée avec lesdites valeurs associées.

3. Procédé selon l'une des revendications précédentes, dans lequel la transformation spatiale calculée (T) est une matrice carrée définissant au moins une rotation et/ou une translation.

4. Procédé selon l'une des revendications précédentes, le procédé comprenant une étape supplémentaire (S31) de réception d'un ensemble de données locales complémentaires par rapport audit organe du patient en provenance d'un dispositif capteur distinct dudit cathéter, et une étape supplémentaire (S33) de correction de la transformation spatiale calculée (T) en fonction de l'ensemble de données locales complémentaires.

5. Procédé selon l'une des revendications précédentes, le procédé comprenant une étape supplémentaire de réception périodique de coordonnées spatiales ($S_{xyz}$), calculées à partir de données reçues de l'au moins un capteur (131) du cathéter (13) à l'intérieur d'une portion (CS) dudit organe du patient ; une étape supplémentaire de modélisation d'un mouvement périodique dudit organe du patient à partir desdites coordonnées spatiales reçues périodiquement et une étape supplémentaire de modification des positions d'affichage du repère anatomique (LM) et du résultat (IH') de la transformation spatiale calculée (T) sur le modèle 3D (IH) sur le dispositif d'affichage de système de navigation (15) en fonction dudit modèle du mouvement périodique dudit organe du patient.

6. Procédé selon l'une des revendications précédentes, le procédé comprenant une étape supplémentaire de calcul d'une valeur (E) par rapport à une erreur de mise en correspondance entre le nuage de points de référence (RPC) et le résultat (IH') de la transformation spatiale calculée (T) sur le modèle 3D (IH).

7. Procédé selon l'une des revendications précédentes, le procédé comprenant une étape supplémentaire (S31) de réception d'un ensemble de données locales complémentaires par rapport audit organe du patient, calculées à partir de données reçues d'au moins un capteur d'un cathéter à l'intérieur d'une portion dudit organe du patient, chacune des données locales complémentaires étant associée à des coordonnées spatiales dudit capteur, et une étape supplémentaire (S41) d'affichage de chacune des données locales complémentaires sur le résultat (IH') de la transformation spatiale calculée (T) sur le modèle 3D (IH) affiché sur le dispositif d'affichage de système de navigation (15), la position de présentation de ces données locales complémentaires étant calculée à partir de leurs coordonnées spatiales associées.

8. Procédé selon la revendication précédente, le procédé comprenant une étape supplémentaire (S42) de calcul, pour chacune des données locales complémentaires, d'une distance entre leurs coordonnées spatiales associées, et un ou plusieurs points du résultat (IH') de la transformation spatiale calculée (T) sur le modèle 3D (IH), et une étape supplémentaire de calcul d'une valeur par rapport à la fiabilité de ces données locales complémentaires à partir de ladite distance calculée.

9. Système de navigation (1) destiné à une assistance d'intervention, le système de navigation comprenant au moins un cathéter (13) comprenant au moins un capteur de positionnement (131), un affichage (15) et une unité de calcul (14) destinée à implémenter le procédé selon l'une des revendications précédentes, et destiné à commander l'affichage dudit repère anatomique (LM) et ledit résultat (IH') de la transformation spatiale calculée (T) sur le modèle 3D (IH) sur ledit affichage.

[Figure 1A]

[Figure 1B]

[Figure 2]

[Figure 3]

[Figure 4]

[Figure 5]

[Figure 6]

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 3622478 A
- US 2018360342 A1

- US 2016331262 A1